# EUROPEAN PATENT SPECIFICATION

(11) **EP 0 783 405 B1**
(45) Date of publication and mention of the grant of the patent: **17.10.2001**
(21) Application number: 95929458.8
(22) Date of filing: 11.08.1995
(51) Int. Cl.: B32B 5/04, B32B 3/24, D04H 13/00

(54) **SLIT ELASTIC FIBROUS NONWOVEN LAMINATES**
GESCHLITZER ELASTISCHER VLIESSCHICHTSTOFF
LAMINES NON TISSES FIBREUX ELASTIQUES AYANT PLUSIEURS FENTES

(30) Priority: 30.09.1994 US 315657
(43) Date of publication of application: 16.07.1997
(73) Proprietor: KIMBERLY-CLARK WORLDWIDE, INC., Neenah, Wisconsin 54956 (US)
(72) Inventor: ABUTO, Frank, Paul, Alpharetta, GA 30202 (US); DIAMOND, Andrew, Edward, Roswell, GA 30076 (US); LEVY, Ruth, Lisa, Sugar Hill, GA 30518 (US); SMITH, Stephen, Clark, Atlanta, GA 30324 (US)
(74) Representative: DIEHL GLAESER HILTL & PARTNER
(86) International application number: US9510174
(87) International publication number: WO9610481

(56) References cited:
- EP-A- 0 312 071
- WO-A-95/29810
- US-A- 4 036 233
- US-A- 4 731 066
- US-A- 5 226 992

## Description

### FIELD OF THE INVENTION

The present invention is directed to elastic fibrous nonwoven laminates. More particularly, the present invention is directed to elastic fibrous nonwoven laminates that are elastic in two directions due to the use of at least one fibrous nonwoven layer which contains a plurality of slits and which is streched while attached to a substrate layer.

### BACKGROUND OF THE INVENTION

Fibrous nonwoven webs are used in an ever increasing number of applications. Examples of such applications include, but are not limited to, workwear and other types of clothing, especially where such products are limited-use and/or disposable. Other applications include health care related items such as medical or surgical drapes, gowns, masks, footwear and headwear and personal care products such as diapers, training pants, incontinence garments, sanitary napkins, bandages and wipers. In many of these and other applications there is often a need for a fibrous nonwoven web which is elastic in nature. By elastic it is meant a material which has a relaxed or first length and which is capable of being stretched or expanded to a second length and then, upon release of the stretching forces, the material is able to retract back to a third length which is equal to or greater than the first length but less than the second length.

There are many examples of fibrous nonwoven webs and laminates which are stretchable and/or elastic. Stretchable materials are distinguishable from elastic materials in that stretchable materials can be expanded in length but they do not necessarily retract back from their expanded length. The methods for making such materials elastic are varied. It is possible to make elastic films and elastic fibrous nonwoven webs. These elastic films and nonwovens often have elastic properties in multiple directions but are also oftentimes lacking in other properties which would make them useful as end use products or components in end use products. As a result, one solution has been to incorporate such elastic materials into laminates. The assignee of record, Kimberly-Clark Corporation, makes a number of materials called stretch-bonded laminates in which one or more gatherable layers are attached at spaced apart points to an elastic layer while the elastic layer is in an expanded state. Once the gatherable layers have been securely attached to the elastic layer, the elastic layer is allowed to relax, thereby causing a plurality of gathers or puckers to form in the outer layer or layers and thus creating a laminate which is stretchable and elastic in at least one direction. In contrast, however, it would be desirable to have an elastic nonwoven laminate which was flatter and devoid of puckers while still having elastic properties.

It is also possible to create laminates which have elastic properties in two directions, however, the processes for forming such materials are significantly more complicated. One method is called neck bonding wherein the outer layers are stretched until they "neck in" in the machine cross direction before they are attached to the elastic interior layer. As a result, the laminate becomes stretchable in the cross machine direction.

From US-A-4,036,233 a disposable diaper is known having a topsheet and a backsheet one of which is stretchable. The other one of the topsheet and backsheet is non-stretchable and both extend beyond the edges of an absorbent material which is sandwiched therebetween to provide a waistband. The non-stretchable sheet is provided with openings to permit stretching of the waistband.

US-A-4,731,066 teaches an elastic laminated disposable diaper with a liquid impermeable backing produced from an initially molten extruded elastic film, an absorbent core sandwiched therebetween and a liquid permeable facing. In the waistband area slits or openings are provided to provide a stretchability to the waistband area.

From EP-A-0 312 071 a diaper article with an elasticized waist panel is known. This article includes a backsheet layer which delimits at least one waistband portion of the article. A liquid permeable topsheet layer is located in facing relation with an inner surface of the backsheet layer and an absorbent body is sandwiched therebetween. An elastic member which is connected to the waistband portion is constructed to shirr the waistband portion for example by the provision of a plurality of slits.

From WO 95/29810, which is a document under Article 54(3) EPC, elastic fibrous nonwoven web laminates are known which exhibit elastic properties in at least one direction due to the use of at least one fibrous nonwoven web facing layer which contains a plurality of slits.

Despite the foregoing processes for forming elastic laminates there is a need for yet additional processes which can quickly and simply create elastic laminates.

### SUMMARY OF THE INVENTION

Disclosed herein is an elastic fibrous nonwoven laminate that is elastic in two directions due to the use of at least one fibrous nonwoven layer which contains a plurality of slits and which has been neck-streched and therefore is expansible in a direction parallel to said slits. Conventional elastomeric nonwoven laminates typically have an elastic layer and a non-elastic layer with the non-elastic layer being bonded to the elastic layer at a plurality of spaced-apart locations while the elastic layer is in a stretched condition. As a result, when the stretching forces are released, the laminate retracts and the non-elastic layer puckers or gathers thereby creating an undulating surface. The present invention uses a nonwoven facing layer containing a plurality of slits and is bonded to an elastic substrate layer while the elastic substrate layer is in a relaxed state. Once the two layers have been laminated to one another, the laminate or composite can be stretched in a direction which is generally perpendicular to the direction of the slits in the nonwoven facing layer. At the same time, because there are no gathers or puckers, the laminate has a flat surface and thus an aesthetically pleasing appearance in both the stretched and unstretched states.

The elastic, fibrous nonwoven laminate includes an elastic substrate layer and a first nonwoven facing layer attached to the elastic substrate layer to form a laminate. The first nonwoven facing layer includes a plurality of slits which are created so that the angle between the longitudinal axis of the slits and the intended direction of strech is between 60° and 120°. The slits in the first nonwoven facing layer can be continuous slits so as to form a plurality of narrow strips of nonwoven facing material or the slits can be discontinuous in a variety of patterns including, but not limited to, an overlapping brick pattern. It is also possible to create discontinuous slits in a number of directions in the nonwoven facing layer. Yet a further alternative is to create slits which are both a combination of continuous and discontinuous slits.

In the most basic configuration, the first nonwoven facing layer is attached to the elastic substrate layer while the elastic substrate layer is in a nonstretched condition to create a two layer laminate. Once the laminate has been formed, it is possible to expand the laminate in a direction which is generally perpendicular to the direction of the slits. Elastic properties in a second direction can be imparted to the laminate by stretching the elastic substrate layer prior to its attachment to the first nonwoven facing layer. Generally this stretching will be in a direction which is parallel to the direction of the slits in the first nonwoven facing layer. As a result, once the two layers have been attached to one another, the first nonwoven facing layer will have a plurality of gathers or puckers which will permit expansion of the laminate in the same direction that the elastic substrate layer was stretched prior to its attachment to the first nonwoven facing layer. The same laminate will also have elastic properties in the other direction due to the expansion of the slits when stretching forces are applied to the laminate in a direction which is generally perpendicular to the direction of the slits. Besides creating a two layer laminate, it is also possible to create a three layer laminate by attaching a second slit fibrous nonwoven facing layer to a surface of the elastic substrate layer which is opposed to the first nonwoven facing layer.

The process for forming such elastic, fibrous nonwoven laminates involves creating a first plurality of slits in a first nonwoven facing layer and then attaching an elastic substrate layer to the first nonwoven facing layer. If desired, a second plurality of slits can be created in a second nonwoven facing layer. This second nonwoven facing layer can then be attached to a surface of the elastic substrate layer which is opposed to the first nonwoven facing layer so as to create a three layer laminate. As still a further process variation, it is possible to stretch the elastic substrate layer and then attach the nonwoven facing layers to the elastic substrate layers while the elastic substrate layer is in a stretched state. As a result, elastic properties can be imparted in two directions with the elastic properties in one direction being dependent upon the formation of the slits in the nonwoven facing layers and the elastic properties in the other direction being dependent upon the stretching of the elastic substrate layer prior to its attachment to the nonwoven facing layers.

Another process for forming an elastic, fibrous nonwoven laminate involves providing a first nonwoven facing layer which has a plurality of slits defined therein. A tensioning force is applied to the first nonwoven facing layer in a direction generally perpendicular to the direction of the slits to neck the first nonwoven facing layer. The plurality of slits may be provided in the first nonwoven facing layer either before or after the layer has been "neck-stretched". The tensioned first nonwoven facing layer containing the plurality of slits is then attached to an elastic substrate layer to form the laminate. If desired, a second nonwoven facing layer also defining a plurality of slits therein may also be neck-stretched and attached to a surface of the elastic substrate layer which is opposed to the tensioned first nonwoven facing layer.

Laminates such as are described above and in further detail below are suitable for a wide variety of the uses mentioned above, not the least of which include components in personal care products including diapers, training pants, incontinence garments, sanitary napkins, bandages and the like.

### BRIEF DESCRIPTION OF THE DRAWINGS

Figure 1 is a perspective view of a slit elastic fibrous nonwoven laminate according to the present invention.

Figure 2 is a top plan view of a slit elastic fibrous nonwoven laminate according to the present invention being stretched along line B-B.

Figure 3 is a top plan view of another slit elastic fibrous nonwoven laminate according to the present invention.

Figure 4 shows the slit elastic fibrous nonwoven laminate of Figure 3 being stretched along line B-B.

Figure 5 is a top plan view of another slit elastic fibrous nonwoven laminate according to the present invention.

Figure 6 shows the slit elastic fibrous nonwoven laminate of Figure 5 being stretched along lines A-A and B-B.

Figure 7 is a perspective view of yet another slit elastic fibrous nonwoven laminate according to the present invention.

Figure 8 is a schematic side view of a process for forming a slit elastic fibrous nonwoven laminate according to the present invention.

Figure 9 is a schematic side view of another process for forming a slit elastic fibrous nonwoven laminate according to the present invention.

Figure 10 is a schematic side view of a process for forming a slit elastic fibrous nonwoven laminate according to the present invention.

Figure 11 is a top plan view of a slit elastic fibrous nonwoven laminate according to the present invention.

Figure 12 is a top plan view of another slit elastic fibrous nonwoven laminate according to the present invention.

### DETAILED DESCRIPTION OF THE INVENTION

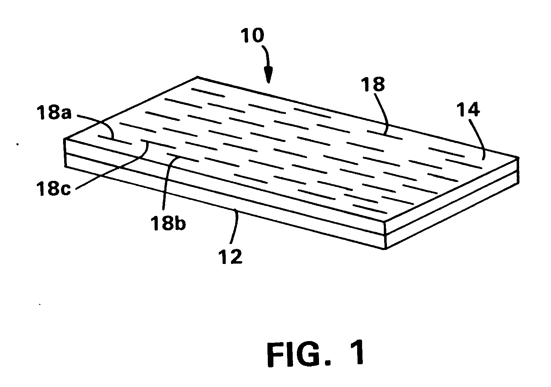

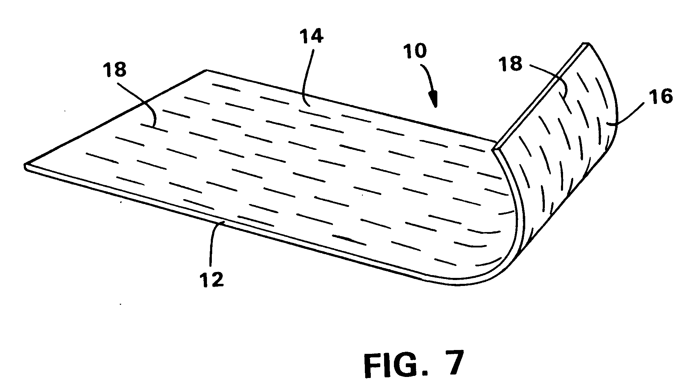

Referring to Figure 1 there is shown an elastic, fibrous nonwoven laminate 10 according to the present invention including an elastic substrate layer 12 and at least a first fibrous nonwoven web facing layer 14. If desired, additional layers may be attached to the laminate 10 as, for example, a second fibrous nonwoven facing layer 16 on a surface of the elastic substrate layer 12 which is opposed to the first facing layer 14. See Figure 7. For purposes of clarity the term "layer" will generally refer to a single piece of material but the same term should also be construed to mean multiple pieces or plies of material which, together, form one or more of the "layers" described herein.

The elastic substrate layer 12 may be made from any material or materials which are elastic in at least one direction and more desirably from materials which are elastic in two or more directions. A material or layer is considered to be "elastic" or have "elastic properties" for purposes of the present invention if it is capable of being stretched or extended from a first and generally relaxed (no external tensional force) length to a second or expanded length which is at least two times the first length and then, upon release of the stretching forces, will retract to a third length which is no greater than 110 percent of the first length or, stated differently, the third length is no greater than 1.1 times the first length. Thus, as an example, a material or layer would be elastic if it had an initial length of 100 centimeters, could be stretched to a length of at least 200 centimeters and then, upon release of the stretching forces, retracted to a length that was no greater than 110 centimeters. For purposes of the present invention, a laminate 10 is said to be "elastic" or have "elastic properties" if the laminate is capable of being stretched from a first length to a second and expanded length which is at least 1.4 times the first length and then, upon release of the stretching forces, will retract to a third length which is no greater than 1.1 times the first length. Thus, as an example, a laminate would have "elastic properties" if it had an initial length of 100 centimeters, could be stretched to a length of at least 140 centimeters and then, upon release of the stretching forces, retracted to a length that was no greater than 110 centimeters.

Suitable elastic materials for the substrate layer 12, include, but are not limited to, elastic threads or yarns, elastic films, elastic nonwoven webs and elastic woven webs as well as combinations of the foregoing. Generally speaking, the elastic or elastomeric webs may be any elastomeric nonwoven fibrous web, elastomeric knitted fabric, elastomeric woven fabric or other elastic material which will exhibit elastic properties. Exemplary elastomeric knitted fabrics are knitted fabrics made utilizing elastomeric threads or yarns which provide stretch and recovery properties in at least one direction. Exemplary elastomeric woven fabrics are fabrics having elastomeric warp and/or weft threads or yarns such as polyurethane threads that provide stretch and recovery properties in at least one direction. Desirably the elastic substrate layer may be made from an elastomeric nonwoven web such as an elastomeric nonwoven web of spunbonded filaments or an elastomeric nonwoven web of meltblown fibers.

Generally, any suitable elastomeric fiber forming resins or blends containing the same may be utilized to form the nonwoven webs of elastomeric fibers of the present invention. For example, useful elastomeric fiber forming resins can include block copolymers having the general formula A-B-A' or A-B, where A and A' are each a thermoplastic polymer endblock which contains a styrenic moiety such as a poly (vinyl arene) and where B is an elastomeric polymer midblock such as a conjugated diene or a lower alkene polymer. Block copolymers of the A-B-A' type can have different or the same thermoplastic block polymers for the A and A' blocks, and these block copolymers are intended to embrace linear, branched and radial block copolymers. In this regard, the radial block copolymers may be designated (A-B)ₘ-X, wherein X is a polyfunctional atom or molecule and in which each (A-B)ₘ-radiates from X in a way such that A is an endblock. In the radial block copolymer, X may be an organic or inorganic polyfunctional atom or molecule and m is an integer having the same value as the functional group originally present in X. It is usually at least 3, and is frequently 4 or 5, but is not limited thereto. Thus, in the present invention, the expression "block copolymer", and particularly "A-B-A'" and "A-B" block copolymer is intended to embrace all block copolymers having such rubbery blocks and thermoplastic blocks as discussed above which can be extruded (e.g., by meltblowing), and without limitation as to the number of blocks. The elastomeric nonwoven web may be formed from, for example, elastomeric (polystyrene/poly(ethylenebutylene)/polystyrene) block copolymers available from the Shell Chemical Company of Houston, Texas under the trade designation KRATON® G. One such block copolymer may be, for example, KRATON® G-1657 copolymer.

Other exemplary elastomeric materials which may be used to form an elastomeric nonwoven web include polyurethane elastomeric materials such as, for example, those available under the trademark ESTANE from B. F. Goodrich & Co., polyamide elastomeric materials such as, for example, those available under the trademark PEBAX from the Rilsan Company, and polyester elastomeric materials such as, for example, those available under the trade designation HYTREL® from E. I. DuPont De Nemours & Company. Formation of an elastomeric nonwoven web from polyester elastomeric materials is disclosed in, for example, U.S. Patent Number 4,741,949 to Morman et al. Elastomeric nonwoven webs may also be formed from elastomeric copolymers of ethylene and at least one vinyl monomer such as, for example, vinyl acetates, unsaturated aliphatic monocarboxylic acids, and esters of such monocarboxylic acids. The elastomeric copolymers and formation of elastomeric nonwoven webs from those elastomeric copolymers are disclosed in, for example, U.S. Patent No. 4,803,117.

Processing aids may be added to the elastomeric polymer. For example, a polyolefin may be blended with the elastomeric polymer (e.g., the A-B-A elastomeric block copolymer) to improve the processability of the composition. The polyolefin must be one which, when so blended and subjected to an appropriate combination of elevated pressure and elevated temperature conditions, is extrudable in blended form with the elastomeric polymer. Useful blending polyolefin materials include, for example, polyethylene, polypropylene and polybutene, including ethylene copolymers, propylene copolymers and butene copolymers. A particularly useful polyethylene may be obtained from the U.S.I. Chemical Company under the trade designation Petrothene NA 601. Two or more of the polyolefins may be utilized. Extrudable blends of elastomeric polymers and polyolefins are disclosed in, for example, U.S. Patent No. 4,663,220 to Wisneski et al.

The elastomeric nonwoven web may also be a pressure sensitive elastomer adhesive web. For example, the elastomeric material itself may be tacky or, alternatively, a compatible tackifying resin may be added to the extrudable elastomeric compositions described above to provide an elastomeric web that can act as a pressure sensitive adhesive, e.g, to bond the elastomeric web to one of the fibrous nonwoven facing layers. In regard to the tackifying resins and tackified extrudable elastomeric compositions, note the resins and compositions as disclosed in U.S. Patent No. 4,787,699 to Kieffer.

Any tackifier resin can be used which is compatible with the elastomeric polymer and which can withstand the high processing (e.g., extrusion) temperatures. If the elastomeric polymer (e.g., A-B-A elastomeric block copolymer) is blended with processing aids, such as for example, polyolefins or extending oils, the tackifier resin should also be compatible with those processing aids. Generally, hydrogenated hydrocarbon resins are preferred tackifying resins because of their better temperature stability. REGALREZ® and ARKON® P series tackifiers are examples of hydrogenated hydrocarbon resins. ZONATAK® 501 lite polymer is an example of a terpene hydrocarbon and is available from Arizona Chemical Company of Wayne, New Jersey. REGALREZ® hydrocarbon resins are available from Hercules Incorporated of Wilmington, Delaware. ARKON® P series resins are available from Arakawa Chemical (U.S.A) Incorporated. Of course, the present invention is not limited to the use of these specific tackifying resins, and other tackifying resins which are compatible with the other components of the composition and which can withstand the high processing temperatures can also be used.

The elastomeric fabric may also be a multilayer material in that it may include two or more individual coherent webs and/or films. Additionally, the elastomeric fabric may be a multilayer material in which one or more of the layers contain a mixture of elastomeric and non-elastomeric fibers or particulates. As an example of the latter type of elastomeric web, reference is made to U.S. Patent No. 4,209,563 to Sisson, in which elastomeric and non-elastomeric fibers are commingled to form a single coherent web of randomly dispersed fibers. Another example of such an elastomeric composite web would be one made by a technique such as is disclosed in U.S. Patent No. 4,741,949 to Morman et al. and U.S. Patent Nos. 4,100,324 to Anderson et al. and 4,803,117 to Daponte. These patents disclose nonwoven materials which include a mixture of meltblown thermoplastic fibers and other materials. Such mixtures may be formed by adding fibers and/or particulates to the gas stream in which elastomeric meltblown fibers are carried so that an intimate entangled commingling of the elastomeric meltblown fibers and other materials occurs prior to collection of the meltblown fibers upon a collection device to form a coherent web of randomly dispersed meltblown fibers and other materials. Useful materials which may be used in such nonwoven elastomeric composite webs include, for example, wood pulp fibers, staple length fibers from natural and synthetic sources (e.g. cotton, wool, asbestos, rayon, polyester, polyamide, glass, polyolefin, cellulose derivatives and the like), non-elastic meltblown fibers, multi-component fibers, absorbent fibers, electrically conductive fibers, and particulates such as, for example, activated charcoal/carbon, clays, starches, metal oxides, superabsorbent materials and mixtures of such materials. Other types of nonwoven elastomeric composite webs may be used. For example, a hydraulically entangled nonwoven elastomeric composite web may be used such as is disclosed in U.S. Patent Nos. 4,879,170 and 4,939,016 both to Radwanski, et al.

If the elastomeric nonwoven web is an elastomeric nonwoven web of meltblown fibers, the meltblown fibers may range, for example, from about 0.1 to about 100 microns in diameter. However, if barrier properties are important in the finished laminate (for example, if it is important that the final laminate material have increased opacity and/or insulating and/or dirt protection and/or liquid repellency), then finer fibers which may range, for example, from about 0.5 to about 20 microns can be used.

The basis weight of the elastomeric fabric may range from about 5 to about 250 grams per square meter. The basis weight can be varied, however, to provide desired properties including recovery and barrier properties, desirably, the basis weight of the elastomeric fabric may range from about 30 to about 100 grams per square meter. Even more particularly, the basis weight of the elastomeric fabric may range from about 35 to about 70 grams per square meter. The extreme thinness of the low basis weight elastomeric nonwoven webs which may be used in certain embodiments of the invention would appear to enhance the material properties of drape and conformability.

In addition to elastic films and nonwovens, elastic wovens also may be used with the present invention. Woven materials are distinguishable from nonwovens given the deliberate and uniform pattern by which the fibers, yarns or filaments are intertwined. Conversely, nonwoven materials are formed from fibers which, at least initially, are laid down in a random pattern and then usually further strengthened by increased entanglement as with hydroneedling and/or bonding of the fibers together.

Besides being elastic, the only other requirement for the substrate layer 12 is that it can be attachable to the facing layers 14 and 16. Where it is desired to have the overall laminate 10 be breathable, it is generally desirable to make the elastic substrate layer from a nonwoven or woven though it is also possible to make films breathable, as, for example, by perforating the films.

Attached to the elastic substrate layer 12 is at least a first fibrous nonwoven web facing layer 14. Generally the facing layer 14 will not be elastic in that it will not meet the requirements of the aforementioned definition of an elastic material prior to being slit. However, the facing layer 14 as with the facing layer 16 may be stretchable, expandable or expansible in that they may be increased in size in one or more directions by applying a tensional force to the material. The "neck-stretched" materials described below are one example of such materials. The basis weight of the facing layer 14 will depend upon the particular end use. The process used to form the fibrous nonwoven web facing layer is left to the discretion of the manufacturer and the design parameters of the overall laminate 10 and/or the particular end product. Generally, it has been found that bonded carded webs and spunbond webs work particularly well as facing layers. The properties of these webs can be further enhanced by forming the webs from all or a portion of multiconstituent and/or multicomponent fibers such as biconstituent and bicomponent fibers. Biconstituent fibers are extruded from a homogeneous mixture of two different polymers. Such fibers combine the characteristics of the two polymers into a single fiber. Bicomponent or composite fibers are composed of two or more polymer types in distinct areas of the fiber such as in a side-by-side or sheath-core configuration.

The processes used to form the fibrous nonwoven web facing layers include those which will result in a material which, as further described below, has the necessary range of physical properties. Suitable processes include, but are not limited to, airlaying, spunbonding and bonded carded web formation processes. Spunbond nonwoven webs are made from fibers which are formed by extruding a molten thermoplastic material as filaments from a plurality of fine capillaries in a spinnerette with the diameter of the extruded filaments then being rapidly reduced, for example, by non-eductive or eductive fluid-drawing or other well known spunbonding mechanisms. The production of spunbonded nonwoven webs is illustrated in patents such as Appel, et al., U.S. Patent 4,340,563; Dorschner et al., U.S. Patent 3,692,618; Kinney, U.S. Patent Numbers 3,338,992 and 3,341,394; Levy, U.S. Patent Number 3,276,944; Peterson, U.S. Patent 3,502,538; Hartman, U.S. Patent 3,502,763 and Dobo et al., U.S. Patent Number 3,542,615.

The spunbond process also can be used to form bicomponent spunbond nonwoven webs as, for example, from side-by-side polyethylene/polypropylene spunbond bicomponent fibers. The process for forming such fibers and resultant webs includes using a pair of extruders for separately supplying both the polyethylene and the polypropylene to a bicomponent spinnerette. Spinnerettes for producing bicomponent fibers are well known in the art and thus are not described herein in detail. In general, the spinnerette includes a housing containing a spin pack which includes a plurality of plates having a pattern of openings arranged to create flow paths for directing the high melting temperature and low melting temperature polymers to each fiber-forming opening in the spinnerette. The spinnerette has openings arranged in one or more rows and the openings form a downwardly extending curtain of fibers when the polymers are extruded through the spinnerette. As the curtain of fibers exit the spinnerette, they are contacted by a quenching gas which at least partially quenches the fibers and develops a latent helical crimp in the extending fibers. Oftentimes the quenching air will be directed substantially perpendicularly to the length of the fibers at a velocity of from about 30 to about 120 meters per minute at a temperature between about 7° and about 32°C.

A fiber draw unit or aspirator is positioned below the quenching gas to receive the quenched fibers. Fiber draw units or aspirators for use in meltspinning polymers are well known in the art. Exemplary fiber draw units suitable for use in the process include linear fiber aspirators of the type shown in U.S. Patent Number 3,802,817 to Matsuki et al. and eductive guns of the type shown in the U.S. Patents 3,692,618 to Dorshner et al. and 3,423,266 to Davies et al. The fiber draw unit in general has an elongated passage through which the fibers are drawn by aspirating gas. The aspirating gas may be any gas, such as air that does not adversely interact with the polymers of the fibers. The aspirating gas can be heated as the aspirating gas draws the quenched fibers and heats the fibers to a temperature that is required to activate the latent crimps therein. The temperature required to activate the latent crimping within the fibers will range from about 43°C to a maximum of less than the melting point of the low melting component polymer which, in this case, is the polyethylene. Generally, a higher air temperature produces a higher number of crimps per unit length of the fiber.

The drawn and crimped fibers are deposited onto a continuous forming surface in a random manner, generally assisted by a vacuum device placed underneath the forming surface. The purpose of the vacuum is to eliminate the undesirable scattering of the fibers and to guide the fibers onto the forming surface to form a uniform unbonded web of bicomponent fibers. If desired, the resultant web can be lightly compressed by a compression roller before the web is subjected to a bonding process.

One way to bond the bicomponent spunbonded web is through the use of a through-air bonder. Such through-air bonders are well known in the art and therefore need not be described herein in detail. In the through-air bonder, a flow of heated air is applied through the web to heat the web to a temperature above the melting point of the lower melting point component of the bicomponent fibers but below the melting point of the higher melting point component. Upon heating, the lower melting polymer portions of the web fibers melt and adhere to adjacent fibers at their cross-over points while the higher melting polymer portions of the fibers tend to maintain the physical and dimensional integrity of the web.

The facing layers also may be made from bonded carded webs. Bonded carded webs are made from staple fibers which are usually purchased in bales. The bales are placed in a picker which separates the fibers. Next, the fibers are sent through a combing or carding unit which further breaks apart and aligns the staple fibers in the machine direction so as to form a generally machine direction-oriented fibrous nonwoven web. Once the web has been formed, it is then bonded by one or more of several bonding methods. One bonding method is powder bonding wherein a powdered adhesive is distributed through the web and then activated, usually by heating the web and adhesive with hot air. Another bonding method is pattern bonding wherein heated calender rolls or ultrasonic bonding equipment are used to bond the fibers together, usually in a localized bond pattern though the web can be bonded across its entire surface if so desired. One of the best methods though, when using bicomponent staple fibers is to use a through-air bonder such as is described above with respect to the bicomponent spunbond web formation process.

In order to obtain the specified range of physical properties of the resultant fibrous nonwoven web according to the present invention, the bonding process used to bond the fibers of the fibrous nonwoven web together should be a process such as through-air bonding which can control the level of compression or collapse of the structure during the formation process. In through-air bonding, heated air is forced through the web to melt and bond together the fibers at their crossover points. Typically the unbonded web is supported on a forming wire or drum. In addition a vacuum may be pulled through the web if so desired to further contain the fibrous web during the bonding process.

Bonding processes such as point bonding and pattern bonding using smooth and/or pattern bonding rolls can be used provided such processes will create the specified range of physical properties for the present invention. Whatever process is chosen, the degree of bonding will be dependent upon the fibers/polymers chosen but, in any event, it is desirable that the amount of web compression be controlled during the heating stage.

Airlaying is another well known process by which fibrous nonwoven webs according to the present invention can be made. In the airlaying process, bundles of small fibers usually having lengths ranging between about 6 and about 19 millimeters are separated and entrained in an air supply and then deposited onto a forming screen, oftentimes with the assistance of a vacuum supply. The randomly deposited fibers are then bonded to one another using, for example, hot air or a spray adhesive.

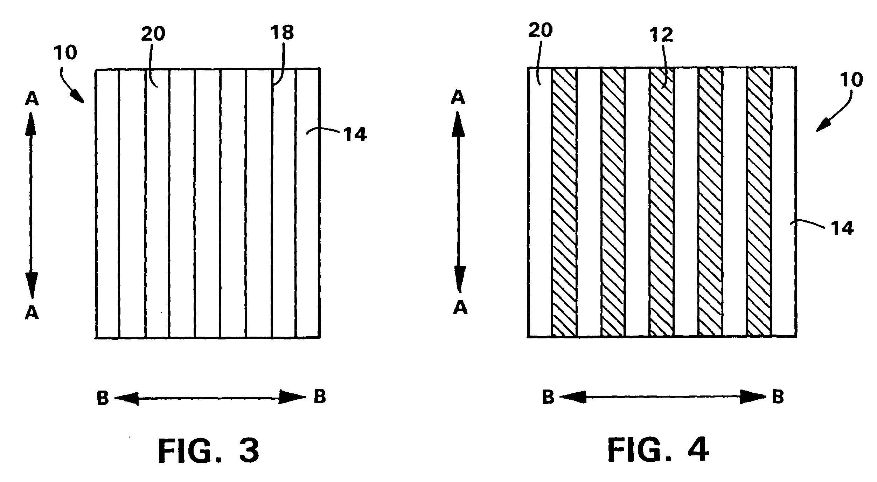

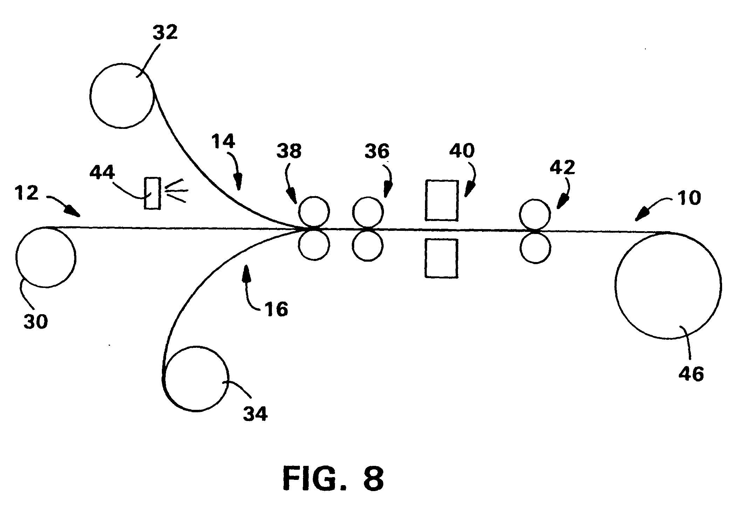

Having described the various components of the laminate 10, a process for forming a laminate 10 according to the present invention is shown in Figure 8. A layer of elastic substrate layer 12 is unrolled from a supply roll 30 and fed through a pair of drive and compaction rolls 36. Alternatively, the elastic substrate layer 12 may be formed directly in-line. Next, a supply of a first fibrous nonwoven web facing layer 14 is unrolled from a supply roll 32 or it also may be formed in-line. Before the facing layer 14 is passed through the drive rolls 36 it must be slit. The slits 18 may be discontinuous such as are shown in Figures 1, 5 and 7 or continuous such as are shown in Figure 3. These slits 18 may be preformed or formed directly in-line as by a slitting roll or other means 38. It is possible to create the slits after the formation of the laminate too. A particularly advantageous slit pattern is one wherein the slits are formed in what is generally referred to as an "overlapping brick pattern." In this pattern the slits in one row overlap the gaps between the slits in an adjacent row. This pattern provides good expansion of the facing layer and the overall laminate. When making continuous slits 18 such as are shown in Figure 3 it is particularly advantageous to perform the slitting in-line just before bonding to the substrate layer 12. Otherwise, handling of the thin strips 20 (See Figure 3) may be difficult.

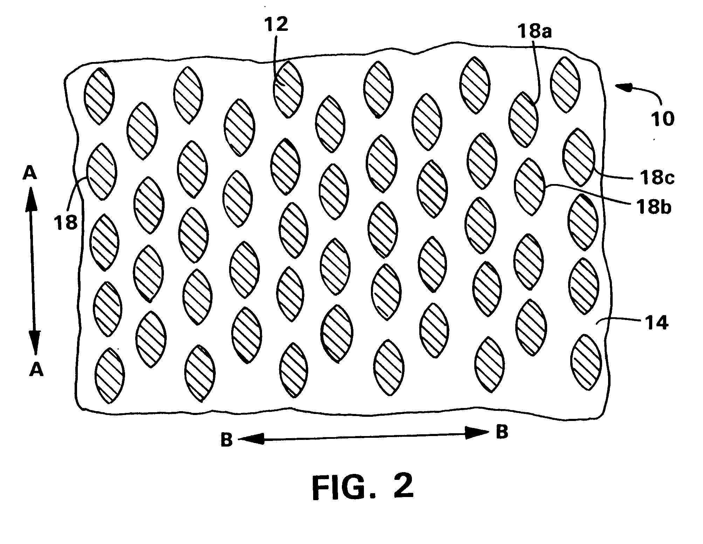

Once the two layers 12 and 14 have been brought together they must be attached to one another. Attachment can be by any suitable means such as heat bonding, ultrasonic bonding, adhesive bonding, needling, stitching or other suitable means. The degree of attachment should be sufficient to maintain attachment during subsequent use of the laminate but not to such a degree as to prevent the slits 18 from opening up in the manner shown in Figures 2, 4 and 6.

As shown in Figure 8, the attachment means in the process includes a heating apparatus 40 for providing hot air and a pair of compaction rolls 42. The surface of the compaction rolls may be smooth and/or patterned. In addition, they may be heated in which case the heating apparatus 40 may be deleted. If a spray adhesive is used, the delivery system 44 must be positioned such that the adhesive is applied to the interior surfaces of the substrate layer 12 and first facing layer 14. Other means for attaching the layers together include but are not limited to ultrasonic bonding, infrared bonding, radio frequency bonding, powdered adhesive bonding, hydroentangling, mechanical entangling such as needling and direct forming of one layer onto another. Once the two layers 12 and 14 have been attached to one another, the resultant laminate 10 may be wound up on a take-up roll 46 or the laminate 10 may remain in-line for further processing.

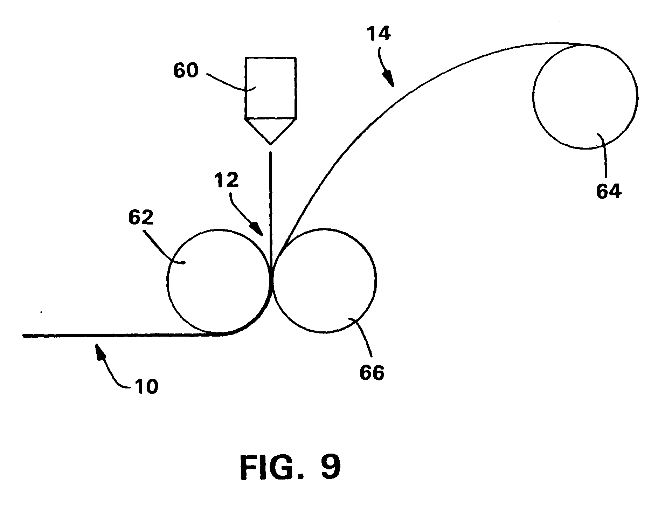

Another process for forming a laminate according to the present invention is shown in Figure 9 of the drawings. In this process the elastic substrate layer 12 is an extruded film emitted from a film die 60. The molten polymer is brought in contact with a chill roll 62 to help solidify the molten polymer. At the same time, a supply 64 of slit nonwoven facing layer material 14 is brought into contact with the still tacky elastic film material 12 between the chill roll 62 and a second roll 66, such as an 85 Shore A rubber roll, which may or may not be chilled. By "chilled" it is meant that the roll 62 or 66 has a temperature which is less than the melting point of the film polymer. As a result of the elastic properties in the film layer 12, a laminate 10 is formed which will at least have elastic properties in the cross-direction (CD) which is along line B-B in Figure 2.

Suitable polymers for forming elastic films include both natural materials (rubber, etc.) and synthetic polymers which will yield a film with elastic properties as defined above. Thus, many of the polymers such as the Kraton® polymers mentioned above with respect to the formation of elastomeric fibers also can be used to form elastomeric films.

As stated at the outset, the elastic substrate layer 12 may have elastic properties in only one direction or in multiple directions. If the elastic substrate layer 12 is only elastic in one direction, then at least a portion of the slits 18 in the facing layer 14 should be generally perpendicular to the direction of elasticity in the elastic substrate layer 12. By "generally perpendicular" it is meant that the angle between the longitudinal axis of the chosen slit or slits and the direction of elasticity is between 60° and 120°. In addition, when it is said that "at least a portion of the plurality of slits must be generally perpendicular to the direction of elasticity or stretch", it is meant that there must be a sufficient number of the described slits which are generally perpendicular such that the overall laminate has "elastic properties". Thus, in Figure 2, if the elastic substrate layer 12 is only elastic in one direction, that direction must be generally along line B-B and not A-A. By placing the direction of elasticity along line B-B, the slits 18 are generally perpendicular to the direction of elasticity. As a result, when stretching forces are applied along line B-B, the slits 18 will open up and permit the laminate 10 to expand in the same direction. Placing the direction of elasticity of substrate 12 along line A-A would not make this possible.

The same rationale also applies to the laminate shown in Figures 3 and 4. Here again if the elastic substrate layer 12 is only elastic in one direction, that direction must be generally aligned with line B-B and not A-A.

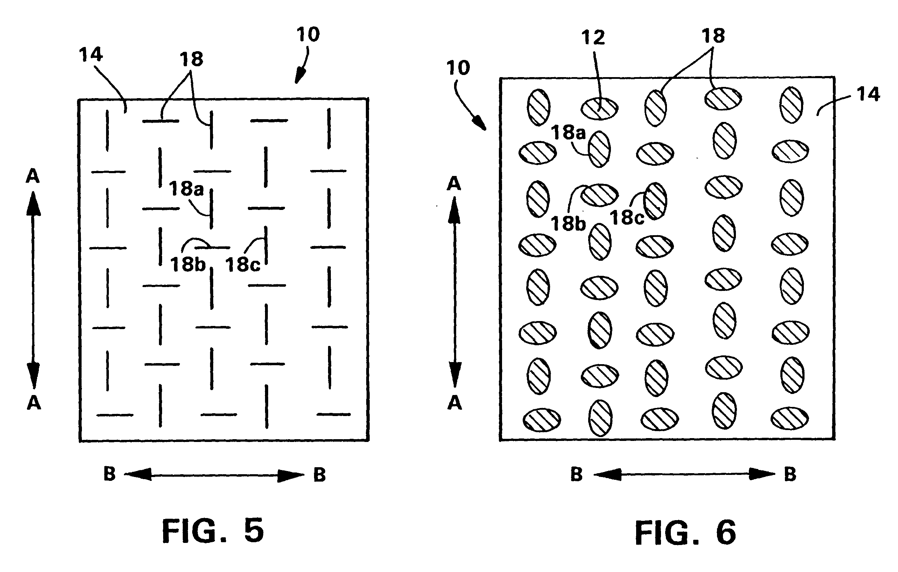

In Figure 5, the fibrous nonwoven facing layer 14 has slits in two directions. One set of slits 18 are generally perpendicular to line A-A while the other set of slits 18 are generally perpendicular to line B-B. This type of slit pattern is particularly advantageous when the elastic substrate layer 12 is elastic in at least two directions as, for example, along lines A-A and B-B. As can be seen from Figure 6, in this configuration, the resultant laminate 10 is capable of exhibiting "elastic properties" in two directions.

In some end use situations, it may be desirable to have an elastic laminate with greater stretch in the machine direction A-A than can be achieved by a plurality of slits 18 perpendicular to the direction A-A in the facing layer 14. In this case, the expansion in the cross machine direction B-B is provided by such slits, generally aligned perpendicular to the direction B-B. However, the desired stretch in the direction A-A is achieved by placing the elastic substrate layer 12 under tension during its attachment to the fibrous nonwoven layer 14. This can be accomplished by driving supply roll 30 and either drive rolls 36 or compaction rolls 42 at different speeds or by braking supply roll 30. In so doing, the elastic substrate layer 12 is stretched in the machine direction. While the elastic substrate layer 12 is in an expanded state, the facing layer 14 is attached to the substrate layer 12, desirably at a plurality of spaced apart locations. Once the two layers have been attached to one another, the tensional forces are removed and the resultant laminate 10 is allowed to retract, thereby forming a plurality of gathers or puckers (not shown) in the facing layer 14. When the resultant laminate 10 is stretched in the machine direction, the elastic substrate layer 12 can be expanded until the slack provided by the gathers or puckers is depleted. When the tensional forces in the machine direction are removed, the elastic substrate layer 12 retracts and the gathers or puckers in the facing layer 14 reappear. A more detailed description of this process can be found in U.S. Patent Number 4,720,415 to Taylor et al.

By applying the tensional forces to the elastic substrate layer 12, elastic properties can be imparted to the laminate 10 in Figures 1 through 4 along lines A-A which is also parallel to the machine direction of the process shown in Figure 8. To impart elastic properties to the laminate 10 in the machine cross direction (along line B-B) the roll 32 of facing layer 14 must be fed into the process of Figure 8 such that the slits 18 are generally parallel to the machine direction of the material (line A-A) and generally perpendicular to the cross direction (line B-B). As a result, the laminate 10 will have elastic properties in the machine direction due to the stretching of the substrate layer 12 during the formation process and elastic properties in the cross direction due to the expandability of the substrate layer 12 and the slits 18 in the facing layer 14.

From the foregoing it can be seen that it is possible to create a two layer laminate 10 which exhibits elastic properties in one or more directions. It is also possible to create multi-layer laminates. For example, the process of Figure 8 can be modified by adding a second fibrous nonwoven facing layer 16 to a surface of the elastic substrate layer 12 which is opposed to the first facing layer 14 to yield a laminate 10 such as is shown in Figure 7. The same processing conditions and techniques can be used to apply the second facing layer 16 to the substrate layer 12 as were described with respect to the first facing layer 14. In addition, it has been found that to maximize the elastic properties of the resultant laminate 10, it is desirable that the slits 18 in the second facing layer 16 be in the same general direction and have the same general pattern as the slits 18 in the first facing layer 14.

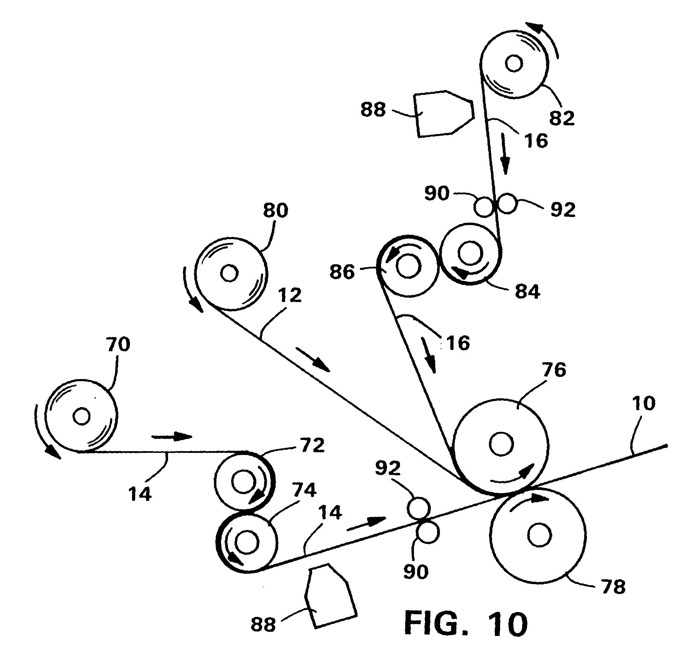

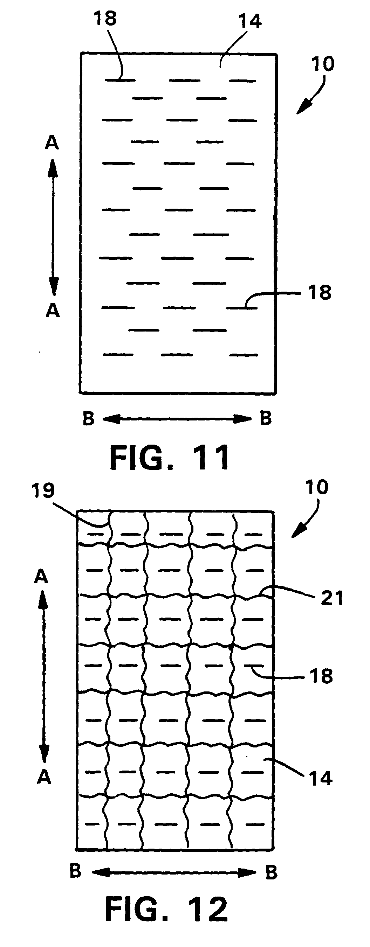

Yet a further embodiment of the present invention is shown in Figures 10 and 11 of the drawings. In this embodiment, the elastic substrate layer 12 is not stretched in any direction, including along line A-A and B-B prior to attachment to the first nonwoven facing layer 14. Instead, the first nonwoven facing layer 14 is "neck-stretched" prior to its being attached to the substrate layer 12. By "neck-stretched" it is meant that the material has been narrowed in at least one direction by the application of a tensioning force.

Referring to Figure 10, a layer of first nonwoven facing layer material 14 is unwound from a supply roll 70 and is passed through a pair of rollers 72 and 74 and from there to a pair of bonding rollers 76 and 78. The rollers 72 and 74 are driven or braked in such a manner that they are traveling at different speeds from one another and with roller 74 being the faster of the two. As a result, as the first fibrous nonwoven facing layer 14 passes through the pairs of rollers 72 and 74, the material 14 is stretched in the machine direction (the direction of travel of the material) and narrowed in the cross-machine direction (the direction perpendicular to the direction of travel of the material). This narrowing or "necking-in" of the material 14 can be maintained through completion of the bonding process to the elastic layer 12. Alternatively, the neck-in configuration of the facing layer material can be locked-in by applying heat to the material to heat set it. This can be accomplished by contacting the material 14 with a heat source 88 such as hot air or an infrared heat source to heat the material and set it.

Once the facing layer material 14 has been neck-stretched and heat set, the tension on the material 14 can be reduced and the material can be slit. To slit the material 14, it is next run through a pair of slitting rolls 90 and 92 to create the desired pattern of slits 18 in the layer 14. Due to the neck stretching, the material 14 is expandable in the direction B-B. The slits which run parallel to the direction B-B cause the material 14 to be expandable in the direction A-A, the machine direction of the material.

As shown in the process of Figure 10, the neck-stretching and slitting take place in-line. It is also possible to form either or both of the functions off-line and then simply feed the pre-slit and neck-stretched material 14 into the bonding rolls 76 and 78. Generally, it is desirable that the slits 18 remain as closed as possible. This is to maximize the ability of the material 14 and the laminate 10 to expand and contract in the machine direction (line A-A). If the material 14 is under too much tension when it is attached to the elastic substrate layer 12, the slits 18 will be too open and thus incapable of sufficient expansion once the laminate 10 has been formed. One way to solve this problem though is to place the substrate layer 12 under the same degree of tension as the facing layer 14 when the two layers enter the bonding rolls 76 and 78. Then, once the two layers 12 and 14 have been bonded together, the tensioning forces can be relaxed and the slits will close back up, thereby making the laminate 10 elastic in the machine direction (line A-A).

At the same time that the facing layer 14 is being neck-stretched and slit, the elastic substrate layer 12 is unwound from supply roll 80 and is fed into the bonding rollers 76 and 78 while under little or no tension. The bonding rollers 76 and 78 or other suitable means of attachment are then used to join the two layers together to form the laminate 10. The resultant laminate 10 is elastic in a direction generally parallel to the direction of neckdown or neck-in (cross-machine direction) of the facing layer 14 and may be stretched in that direction (along line B-B of Figure 11) to the breaking point of the necked material. Due to the elastic properties of the substrate layer 12, once the laminate 10 has been stretched in the cross-machine direction (along line B-B of Figure 11), the laminate 10 will retract back. As a result of the slits 18, the laminate 10 will also have elastic properties in the machine direction or along line A-A in Figure 11. The making of neck-stretched materials is explained in further detail in U.S. Patent Nos. 5,226,992 to Morman, 5,320,891 to Levy et al. and 5,114,781 to Morman.

As with the other laminate 10, it is also possible to attach a second fibrous nonwoven facing layer 16 to a side of the elastic substrate layer 12 opposite that of the first fibrous nonwoven facing layer 14. Referring again to Figure 10, the second facing layer 16 in unwound from a second supply roll 82 and fed into a pair of rollers 84 and 86 in the same fashion as the first facing layer 14. It too is neck-stretched by braking or driving rollers 84 and 86 at different speeds with respect to one another. As a result, the second facing layer 16 is necked-in and then bonded to the substrate layer 12 in the same fashion as the first facing layer 14. Again, to impart elastic properties in the machine direction, the second layer 16 is provided with a plurality of slits which are generally parallel to those in the first facing layer 14.

A further embodiment of the present invention is shown in Figure 12 of the drawings. In this embodiment the laminate 10 is a single layer of the neck-stretched material 14 shown in Figure 11 and made via the process shown in Figure 10. The neck-stretched material 14 has a plurality of slits 18 which are generally perpendicular to the machine direction of the material designated by line A-A and parallel to the direction of cross-machine direction stretch imparted by the neck stretching (line B-B). As with the other embodiments, the material 14 can be stretched in direction A-A due to the slits 18 but because there is no elastic layer 12, the layer 14 will not retract back. Consequently, layer 14 is attached to an elastic substrate layer which comprises a plurality of elastic threads 19 which are generally perpendicular to the direction of the slits 18. As a result, the layer 14 can be stretched or expanded in the direction A-A due to the slits 18 and will retract back due to the elasticity imparted by the stretched elastic threads 19. In the same fashion, the neck-stretching will permit stretching or expansion of layer 14 in the direction B-B and retraction capability is provided by a second plurality of elastic threads 21 attached to layer 14.

The elastic threads 19 and 21 may be attached to the facing layer 14 in a number of ways which include, but are not limited to, stitching, sewing and gluing. It also may be desirable to design the slit pattern and plan the location of attachment of the threads 19 and 21 such that they do not bridge the slits 18. See Figure 12.

Based upon the below examples and testing it was found that embodiments in which the facing layers 14 and 16 utilized discontinuous slits such as are shown in Figures 1 and 7 tended to work better than the continuous slit versions of the present invention such as are shown in Figures 3 and 4. Generally the nonwoven facing layers will have basis weights ranging from about 12 grams per square meter to about 210 grams per square meter with more defined ranges based upon specific end uses including from about 34 to about 100 grams per square meter and from about 50 to about 70 grams per square meter. The bicomponent fibers and especially the through-air bonded spunbond versions seemed to work quite well due to their ability to bond to one another. Generally the fiber sizes will be less than about 6 denier while in specific applications fiber sizes may be less than 3.5 denier or even 2.5 denier and below. The overlapping brick pattern of slitting such as is shown in Figures 1 and 2 seemed to work particularly well. In such configurations, the length of the slits typically will range between about 3 and about 50 millimeters and the distance between aligned slits in direction A-A as, for example, 18a and 18b will be less than 50 millimeters and often they will be less than 20 millimeters and in some cases less than 10 millimeters. In the direction B-B, the distance between any two adjacent slits as, for example, 18b and 18c will be less than 50 millimeters and generally less than 10 millimeters or even less than 5 millimeters. The basis weight of the elastic substrate layer can vary greatly depending upon the particular end use though, generally, the basis weight will be less than 250 grams per square meter and generally less than 100 grams per square meter and oftentimes even less than 50 grams per square meter.

From a processing standpoint when using elastic films, the process in Figure 9 works very well. The lamination of the facing layers to the elastic substrate layer occurs in the nip while the elastic film substrate is in a semi-molten state and therefore tacky enough to provide the desired adhesion strength between the facing layers and the elastic substrate. The elastic laminate so produced will generally have a basis weight less than about 700 grams per square meter and generally less than 300 grams per square meter and oftentimes even less than 150 grams per square meter.

The elastic substrate layer itself can be laminated layers as can be the nonwoven facing layer. The outer facing layers can be used to cover the elastic substrate and impart aesthetic or protective features (abrasion resistance). These outer facings can also impart a stretch-to-stop feature. Stretch-to-stop can be important in protecting the composite from tensile failure due to overextension.

Having described the materials and process of the present invention, several sample laminates are set forth below to further illustrate the present invention. It should be understood, however, that these examples are illustrative only and are not meant to limit the breadth and scope of the present invention.

### EXAMPLE 1

In example 1, an elastic, two layer fibrous nonwoven laminate was made using a process similar to that shown in Figure 9 of the drawings. The elastic substrate layer was a 69 gram per square meter elastic film extruded from a KRATON® G 2755 elastomer made by the Shell Chemical Company of Houston, Texas. The substrate layer had elastic properties in both direction A-A and B-B depicted in Figure 2. To the elastic film substrate layer there was laminated a 100 gram per square meter through-air bonded side-by-side polypropylene /polyethylene two denier bicomponent spunbond facing layer. The facing layer contained a plurality of slits in an overlapping brick pattern as shown, for example, in Figures 1 and 2. The slits were 9.5 millimeters in length and there was 6.35 millimeters between any two aligned slits in direction A-A of Figure 2 and 3.2 millimeters in direction B-B. The facing layer and elastic film substrate layer were laminated to one another in a nip comprising a chill roll and an 85 Shore A rubber roll. The elastic film layer had just been formed and still had sufficient tack to provide the necessary degree of attachment to the facing layer. The resultant laminate had elastic properties in the cross-direction or along line B-B. The laminate had a first length of 8.5 centimeters, a fully stretched second length of 16 centimeters and a third length of 9 centimeters.

### EXAMPLE 2

In Example 2, the same fibrous nonwoven facing layer was used as was used in Example 1. The elastic substrate layer in Example 2 was an elastomeric fibrous meltblown nonwoven web having a basis weight of 196 grams per square meter. The meltblown web was made from KRATON® G 2740 manufactured by the Shell Oil Company of Houston, Texas. The elastic meltblown web had elastic properties in both the A-A and B-B directions such as are shown in Figure 2. To create stretch in the overall laminate in both the machine direction (A-A) and in the cross-direction (B-B) the elastic meltblown web was stretched in the machine direction prior to its attachment to the fibrous nonwoven facing layer. Attachment of the two layers was achieved through the use of 7 grams per square meter of a spray adhesive applied to the interior surface of one or the other of the two layers. Once the two layers had been joined to one another the laminate had elastic properties in both the machine and cross directions. In the cross-direction (B-B), the laminate had a first length of 7.5 centimeters, a fully stretched second length of 15.5 centimeters, and a recovered third length of 7.9 centimeters. In the machine direction (A-A), the laminate had a first length of 11 centimeters, a fully stretched second length of 15.8 centimeters and a third length of 11.4 centimeters.

### EXAMPLE 3

In Example 3, the fibrous nonwoven facing layer was identical to the facing layer used in Examples 1 and 2 but for the basis weight. In Example 3, the basis weight of the bicomponent spunbond web was 97.6 grams per square meter versus the previous basis weight of 100 grams per square meter. The slit pattern and its dimensions were the same as that used in the previous examples. The elastic substrate layer was also the same as that used in Example 2 but for the basis weight. In Example 3, the basis weight of the elastomeric meltblown nonwoven web was 74.7 grams per square meter. In this example, while the elastic nonwoven web had elastic properties in both the A-A and B-B directions, it was not prestretched prior to its attachment to the fibrous nonwoven facing layer. As with Example 2, a spray adhesive was applied to one of the two layers in the amount of 7 grams per square meter and the two layers were adhesively laminated to one another. The elastic nonwoven laminate had a first length in direction B-B of 8.3 centimeters, a second fully stretched length of 20.4 centimeters and a recovered third length of 8.8 centimeters.

### EXAMPLE 4

In Example 4 another cross direction stretchable material was formed. The nonwoven facing layer was again made from the same spunbond material as in the previous examples. It had a basis weight of 20.3 grams per square meter and the same slit pattern and dimensions as previously described. The elastic substrate layer was the same 196 gram per square meter elastic nonwoven meltblown web described in Example 2. The two layers were attached to one another using heat and pressure. The facing layer was attached to the elastic substrate layer in a Carver Model 2518 laboratory press from Fred S. Carver Inc. of Menomonee Falls, Wisconsin at a pressure of 30 pounds per square inch (2.07 x 10⁵ Newtons per square meter) and a temperature of 49°C. The elastic laminate had a first relaxed length of 9.6 centimeters in the cross machine direction (B-B), a fully stretched second length of 20.5 centimeters and a third recovered length of 10 centimeters.

### EXAMPLE 5

In Example 5, two 100 gram per square meter through-air bonded, side-by-side polypropylene/polyethylene 2.0 denier bicomponent facing layers were laminated to either side of an elastic meltblown substrate layer. The two exterior layers were the same materials described in Example 1 and the elastic meltblown substrate layer was the same material and basis weight as that described in Example 2. No stretching was imparted to the elastic substrate layer in the machine direction during application of the nonwoven facing layers. Instead, the two facing layers were applied to either side of the elastic substrate layer using 7 grams per square meter of spray adhesive applied to both sides of the substrate layer in an overall spray pattern. The slits in the first and second facing layers were aligned in the same direction with one another. The laminate had a relaxed first length in the cross-direction (B-B) of 14 centimeters, a second fully stretched length of 22.5 centimeters and a third recovered length of 14.5 centimeters.

### EXAMPLE 6

In Example 6, an elastic, fibrous nonwoven laminate was made using an 84 gram per square meter hydroentangled pulp fiber/spunbond fiber composite. The composite included a mixture of 14 grams per square meter of 3.0 denier polypropylene fibers and 70 grams per square meter of wood pulp fibers. Such a hydroentangled composite can be made in accordance with the teachings of U.S. Patent Number 5,284,703 to Everhart et al. The facing layer contained continuous slits such as are shown in Figure 3. It was found that the pulp content in the composite made it easier to slit while the hydroentangling process improved the cloth-like appearance. The slit facing layer was adhesively attached to a 118 gram per square meter KRATON® G 2740 meltblown substrate layer which had elastic properties in both the A-A and B-B directions. The strips of facing layer were continuous in direction A-A and were from 3 to 5 millimeters in width in the direction of B-B. The resultant laminate stretched in direction B-B (See Figure 4) and had a relaxed first length of 14.9 centimeters, a second fully stretched length of 27.0 centimeters and a third recovered length of 15.7 centimeters.

### EXAMPLE 7

In Example 7, an elastic, fibrous nonwoven laminate was made using a 50 gram per square meter through-air bonded side-by-side polypropylene/polyethylene two denier bicomponent spunbond facing layer. The facing layer contained a plurality of slits that were generally 10 mm but ranged from 8-15 mm in length in a pattern similar to that shown in Figures 5 and 6. The spacing between any two adjacent slits in directions A-A and B-B ranged from 8-30 millimeters. To the slit facing layer there was attached a 60 gram per square meter KRATON® G 2755 elastic film using 80 grams per square meter of spray adhesive to perfect the attachment. The elastic film had elastic properties in both directions A-A and B-B. As a result, the laminate also had elastic properties in both directions. In the direction A-A, the sample had a first relaxed length of 8.0 centimeters, a second fully strength length of 14.0 meters and a third recovered length of 8.4 centimeters. In the direction B-B, the same sample had first, second and third lengths of 8.5 centimeters, 12.5 centimeters and 8.8 centimeters respectively.

### EXAMPLE 8

In Example 8, an elastic, fibrous nonwoven laminate having two facing layers was made using two 205 gram per square meter (6 ounces per square yard) through-air bonded, side-by-side polyethylene/polypropylene two denier bicomponent spunbond facing layers. The facing layers contained a plurality of 10 to 15 millimeter long slits in an overlapping brick pattern. The spacing between two adjacent slits in direction A-A and B-B was 5 millimeters. In between the two slit facing layers there was attached a 34 gram per square meter meltblown elastomeric nonwoven web made from Arnitel® EM 400 copolyetherester polymer from DSM Engineering Plastics. Such elastomeric meltblown webs can be made in accordance with the teachings of U.S. Patent Number 4,707,398 to Boggs and U.S. Patent Number 4,741,941 to Morman et al. The meltblown elastic nonwoven substrate layer had elastic properties in both the A-A and B-B directions. Each of the slit facing layers and the elastic substrate layer were bonded to one another using 10 grams per square meter of a spray adhesive. The laminate extended to 1.5 times its original length when stretched and returned to its original length when the stretching forces were relaxed.

### EXAMPLE 9

In Example 9 the nonwoven facing layers were the same as those used in Example 8. The slit nonwoven facing layers were attached to an elastic laminate substrate layer which included the same 34 gram per square meter meltblown elastic substrate layer from Example 8 which had been previously laminated to a 137 gram per square meter (4.0 ounces per square yard) necked-in polypropylene spunbond nonwoven web. The laminated substrate layer was adhesively attached to each of the two slit nonwoven facing layers using 10 grams per square meter of spray adhesive. The unique feature of this embodiment was the stretch-to-stop feature of the laminate substrate layer. More specifically, the substrate layer could only be stretched a predetermined distance which was governed by the full expansion of the spunbond portion of the laminated substrate layer. Once this layer had been completely stretched, the laminate would stop stretching. As a result, the overall laminate could be specifically designed so as to prevent the slit nonwoven facing layers from overstretching which in turn could cause tares and/or delamination of the overall laminate. The overall laminate exhibited elastic properties.

### EXAMPLE 10

In Example 10, a fibrous nonwoven laminate with stretched-to-stop functionality similar to that in Example 9 was made using the same bicomponent spunbond facing layer material of Example 9 sandwiched between and adhesively bonded to two layers of substrate layer material. The two pieces of substrate layer material were each made from a 34 gram per square meter meltblown elastic nonwoven web such as was previously described with respect to Examples 8 and 9. This elastic meltblown web was laminated to a tricot knit fabric style 850 from Mantex Fabric Corporation of New York City, New York. These two elastic meltblown/woven laminate substrate layers were adhesively attached to both sides of the slit bicomponent spunbond using 10 grams per square meter of spray adhesive on both sides of the bicomponent spunbond and with the elastic meltblown layers of the two laminates facing the slit bicomponent layer. As with Example 9, stretch-to-stop characteristics were imparted to the overall composite via to the woven components in the two substrate layers. The laminate when stretched exhibited elastic properties.

As can be seen in all the examples described above, an elastic, fibrous nonwoven laminate was formed which in all cases had elastic properties in the cross machine direction and, as shown in Examples 2 and 7, a laminate could also be created which had elastic properties in both the machine and cross directions. As a result, the present invention can be used to create elastic laminates which can be used in a wide variety of applications not the least of which includes personal care absorbent products such as diapers, training pants, incontinence garments, sanitary napkins, bandages and the like.

### EXAMPLE 11

In Example 11 an elastic, two layer fibrous nonwoven laminate according to the present invention was made using a 51 gram per square meter spunbond-meltblown-spunbond (SMS) facing layer. The three layers of the SMS laminate were of equal basis weight. The meltblown nonwoven layer was made from polypropylene and each of the spunbond layers were made from a copolymer containing 97% polypropylene and 3% polyethylene. The SMS material was neck-stretched by subjecting the material to tension in the direction of travel. Subsequently, the material was heat set at a temperature of 120°C for a period of approximately 20 seconds. The resultant neck-stretched material had a basis weight of 63 grams. The neck-stretched material was reduced in width by 40% and it was able to expand in the direction B-B depicted in Figure 11. A sample of the necked-in facing layer had a first length of 25.4 centimeters, a fully expanded second length of 35.6 centimeters and a final relaxed length of 26.7 centimeters in the direction B-B of Figure 11. It had no elastic properties in the direction A-A. To impart elastic properties to the laminate in the machine direction A-A, a plurality of slits were provided to the facing layer in a direction B-B generally perpendicular to the machine direction. The slits were 9.5 millimeters in length and there was 6.4 millimeters between any two aligned slits in direction B-B of Figure 11 and 3.2 millimeters in direction A-A. The facing layer with slits was attached to an elastic substrate through the use of 10.7 grams per square meter spray adhesive applied to the interior surface of one or the other of the two materials to produce the elastic fibrous nonwoven laminate. The elastic substrate layer in Example 11 was a nonwoven meltblown web comprising integrated elastomeric and polypropylene fibers with a combined basis weight of 85 grams per square meter. Approximately 89% of the meltblown web was extruded from KRATON® G 2755 elastomer manufactured by Shell Chemical Company of Houston, Texas and the remaining 11% was extruded from Himont PF-015 polypropylene polymer made by Himont, USA of Wilmington, Delaware. The elastic substrate had elastic properties in both directions A-A and B-B of Figure 11. Once the two layers had been joined to one another, the resulting fibrous nonwoven laminate also had elastic properties in both the machine and cross-directions. In the cross-direction (B-B), the laminate had a first length of 25.4 centimeters, a fully stretched second length of 35.6 centimeters and a final recovered length of 25.4 centimeters. In the machine direction (A-A), the laminate had a first length of 16.5 centimeters, a fully stretched second length of 25.4 centimeters and a final recovered length of 17.1 centimeters.

### EXAMPLE 12

In Example 12, the same neck-stretched, SMS facing layer with the same slit configuration was used as that described in Example 11. To one side of this neck-stretched facing layer there was attached a plurality of elastic threads in an overlapping pattern with the spacing of the threads being approximately one-half inch (1.27 cm). A first plurality of threads was aligned in the general direction of line A-A and a second plurality of threads was positioned along line B-B. The elastic threads were attached to the neck-stretched facing layer using a spray adhesive and were attached to the facing layer while in a non-stretched configuration.

## Claims

1. A process for forming an elastic, fibrous nonwoven laminate (10), comprising:
creating a first plurality of slits (18) in a first nonwoven facing layer (14), wherein at least a portion of said first plurality of slits (18) is created in a direction so that the angle between the longitudinal axis of the slits and the intended direction of stretch of said laminate (10) is between 60° and 120°, applying a tensioning force to said first nonwoven facing layer (14) in a direction perpendicular to the direction of said slits (18) to neck said first nonwoven facing layer (14); and
attaching an elastic substrate layer (12) to said tensioned first nonwoven facing layer (14).

2. The process of claim 1 which further includes the step of creating a second plurality of slits in a second nonwoven facing layer (16) and;
attaching said second nonwoven facing layer (16) to a surface of said elastic substrate layer (12) which is opposed to said first nonwoven facing layer (14).

3. The process of claim 1 which further includes the step of stretching said elastic substrate layer (12) and attaching said first nonwoven facing layer (14) to said elastic substrate layer (12) while said elastic substrate layer (12) is in a stretched state.

4. The process of claim 1 wherein said first plurality of slits (18) are formed into said first nonwoven facing layer (14) in an overlapping brick pattern.

5. The process of claim 2, wherein a tensioning force is applied to said second nonwoven facing layer (16) in a direction perpendicular to the direction of said slits (18) to neck said second nonwoven facing layer (16) and said tensioned second nonwoven facing layer (16) is attached to a surface of said elastic substrate layer (12) which is opposed to said tensioned first nonwoven facing layer (14).

6. An elastic, fibrous nonwoven laminate (10) comprising an elastic substrate layer (12) and a first nonwoven facing layer (14) attached to said elastic substrate layer (12) to form a laminate, said first nonwoven facing layer (14) defining a plurality of slits (18) therein, wherein at least a portion of said first plurality of slits (18) is created in a direction so that the angle between the longitudinal axis of the slits and the intended direction of stretch of said laminate (10) is between 60° and 120°, said laminate (10) having elastic properties in a direction which is perpendicular to a direction of at least a portion of said plurality of slits, and wherein said laminate (10) has additional elastic properties in a direction which is not perpendicular to said direction of at least a portion of said plurality of slits (18), **characterized in that** said first nonwoven facing layer (14) having been neck-stretched and therefore being expansible in a direction parallel to said slits (18).

7. The elastic fibrous nonwoven laminate (10) of claim 6 which further includes a second nonwoven facing layer (16) attached to a surface of said elastic substrate layer (12) which is opposed to said first nonwoven facing layer (14).

8. The elastic, fibrous nonwoven laminate (10) of claim 6 where said slits (18) are in an overlapping brick pattern.

9. The elastic, fibrous nonwoven laminate (10) of claim 6 wherein said laminate (10) forms at least a portion of a personal care absorbent product.

10. The elastic, fibrous nonwoven laminate (10) of claim 6 wherein said laminate (10) is in the form of a medical drape.

11. The elastic, fibrous nonwoven laminate (10) of claim 6 wherein said laminate (10) forms at least a portion of an article of clothing.

12. The elastic fibrous nonwoven laminate (10) of claim 7 wherein said second nonwoven facing layer (16) has been neck-stretched and therefore being expansible in a direction parallel to said slits (18).

13. The elastic, fibrous nonwoven laminate (10) of claim 12 wherein said elastic substrate layer (12) comprises a first plurality of elastic threads which are created in a direction so that the angle between the longitudinal axis of the slits and the longitudinal axis of the elastic threads is between 60° and 120°, and a second plurality of elastic threads which are parallel to said slits (18).

## Patentansprüche

1. Verfahren zum Bilden eines elastischen Faservlieslaminats (10), umfassend:
Erzeugen einer ersten Mehrzahl von Schlitzen (18) in einer ersten Vliesauflageschicht (14), wobei wenigstens ein Teil der ersten Mehrzahl von Schlitzen (18) in eine Richtung erzeugt wird, so dass der Winkel zwischen der Längsachse der Schlitze und der vorgesehenen Dehnrichtung des Laminats (10) zwischen 60° und 120 beträgt
Anlegen einer Dehnkraft an die erste Vliesauflageschicht (14) in einer Richtung senkrecht zur Richtung der Schlitze (18), um die erste Vliesauflageschicht (14) zu strecken; und
Aufbringen einer elastischen Substratschicht (12) auf die gedehnte erste Vliesauflageschicht (14).

2. Verfahren gemäß Anspruch 1, welches ferner den Schritt des Erzeugens einer zweiten Mehrzahl von Schlitzen in einer zweiten Vliesauflageschicht (16) umfasst, und;
Aufbringen der zweiten Vliesauflageschicht (16) auf eine Oberfläche der elastischen Substratschicht (12), gegenüber der ersten Vliesauflageschicht (14).

3. Verfahren gemäß Anspruch 1, welches ferner den Schritt des Dehnens der elastischen Substratschicht (12) und des Aufbringens der ersten Vliesauflageschicht (14) auf die elastische Substratschicht (12) während die elastische Substratschicht (12) sich in einem gedehnten Zustand befindet, umfasst.

4. Verfahren gemäß Anspruch 1, wobei die erste Mehrzahl von Schlitzen (18) in der ersten Vliesauflageschicht (14) in einem überlappenden Ziegelmuster gebildet wird.

5. Verfahren gemäß Anspruch 2, wobei eine Dehnkraft an die zweiten Vliesauflageschicht (16) in eine Richtung senkrecht zu der Richtung der Schlitze (18) angelegt wird, um die zweite Vliesauflageschicht (16) zu strecken, und wobei die gedehnte zweite Vliesauflageschicht (16) auf eine Oberfläche der elastischen Substratschicht (12) gegenüber der gespannten ersten Vliesauflageschicht (14) aufgebracht wird.

6. Elastisches Faservlieslaminat (10) umfassend eine elastische Substratschicht (12) und eine auf die elastische Substratschicht (12) aufgebrachte erste Vliesauflageschicht (14), wobei ein Laminat gebildet wird, wobei die erste Vliesauflageschicht (14) eine Mehrzahl von Schlitzen (18) darin definiert, wenigstens ein Teil der Mehrzahl von Schlitzen (18) in eine Richtung erzeugt ist, so dass der Winkel zwischen der Längsachse der Schlitze und der vorgesehenen Dehnrichtung des Laminates (10) zwischen 60° und 120° beträgt, wobei das Laminat (10) elastische Eigenschaften in einer Richtung senkrecht zu einer Richtung von wenigstens einem Teil der Mehrzahl von Schlitzen aufweist, und wobei das Laminat (10) zusätzliche elastische Eigenschaften aufweist, in einer Richtung, die nicht senkrecht zur Richtung von wenigstens einen Teil der Mehrzahl von Schlitzen (18) quergestreckt ist, **dadurch gekennzeichnet, dass** die erste Vliesauflageschicht (14) quergestreckt ist und somit in einer Richtung parallel zu den Schlitzen (18) ausdehnbar ist.

7. Elastisches Faservlieslaminat (10) gemäß Anspruch 6, welcher ferner eine zweite Vliesauflageschicht (16) umfasst, die auf einer Oberfläche der elastischen Substratschicht (12) gegenüber einer ersten Vliesauflageschicht (14) aufgebracht ist.

8. Elastisches Faservlieslaminat (10) gemäß Anspruch 6, wobei die Schlitze (18) in einem überlappenden Ziegelmuster vorliegen.

9. Elastisches Faservlieslaminat (10) gemäß Anspruch 6, wobei das Laminat (10) wenigstens einen Teil eines saugfähigen Hygieneprodukts bildet.

10. Elastisches Faservlieslaminat (10) gemäß Anspruch 6, wobei das Laminat (10) die Form eines medizinischen Stoffes aufweist.

11. Elastisches Faservlieslaminat (10) gemäß Anspruch 6, wobei das Laminat (10) wenigstens einen Teil eines Bekleidungsstückes bildet.

12. Elastisches Faservlieslaminat (10) gemäß Anspruch 7, wobei die zweite Vliesauflageschicht (16) quergestreckt ist und daher in eine Richtung parallel zu den Schlitzen (18) ausdehnbar ist.

13. Elastisches Faservlieslaminat (10) gemäß Anspruch 12, wobei die elastische Substratschicht (12) eine erste Mehrzahl von elastischen Fäden, die in eine Richtung erzeugt sind, so dass der Winkel zwischen der Längsachse der Schlitze und der Längsachse der elastischen Fäden zwischen 60° und 120° beträgt, und eine Mehrzahl von elastischen Fäden parallel zu den Schlitzen (18), umfasst.

## Revendications

1. Procédé de formation d'un stratifié (10) élastique, fibreux, non tissé, comprenant :
la création d'une première série de fentes (18) dans une première couche de façade non tissée (14), au moins une partie de ladite première série de fentes (18) étant créée selon une direction telle que l'angle entre l'axe longitudinal des fentes et la direction voulue d'extensibilité dudit stratifié (10) est compris entre 60° et 120°
l'application d'une force de traction à ladite première couche de façade non tissée (14) selon une direction perpendiculaire à la direction desdites fentes (18) pour provoquer une striction dans ladite première couche de façade non tissée (14) ; et
la fixation d'une couche de substrat élastique (12) à ladite première couche de façade non tissée (14) sous tension.

2. Procédé selon la revendication 1, qui comprend en outre l'étape de création d'une seconde série de fentes dans une seconde couche de façade non tissée (16) ; et
la fixation de ladite seconde couche de façade non tissée (16) à une surface de ladite couche de substrat élastique (12) opposée à ladite première couche de façade non tissée (14).

3. Procédé selon la revendication 1, qui comprend en outre l'étape d'étirage de ladite couche de substrat élastique (12) et la fixation de ladite première couche de façade non tissée (14) à ladite couche de substrat élastique (12) tandis que ladite couche de substrat élastique (12) est à l'état étiré.

4. Procédé selon la revendication 1, dans lequel ladite première série de fentes (18) est formée dans ladite première couche de façade non tissée (14) selon un motif en quinconce avec zones de recouvrement.

5. Procédé selon la revendication 2, dans lequel une force de traction est appliquée à ladite seconde couche de façade non tissée (16) selon une direction perpendiculaire à la direction desdites fentes (18) pour provoquer une striction dans ladite seconde couche de façade non tissée (16), et dans lequel ladite seconde couche de façade non tissée (16) sous tension est fixée à une surface de ladite couche de substrat élastique (12) opposée à ladite première couche de façade non tissée (14) sous tension.

6. Stratifié (10) élastique, fibreux, non tissé, comprenant une couche de substrat élastique (12) et une première couche de façade non tissée (14) fixée à ladite couche de substrat élastique (12) pour former un stratifié, ladite première couche de façade non tissée (14) définissant en son sein une série de fentes (18), stratifié dans lequel au moins une portion de ladite première série de fentes (18) est créée selon une direction telle que l'angle compris entre l'axe longitudinal des fentes et la direction voulue d'extensibilité dudit stratifié (10) est compris entre 60° et 120°, ledit stratifié (10) ayant des propriétés élastiques dans une direction qui est perpendiculaire à une direction d'au moins une partie de ladite série de fentes, et dans lequel ledit stratifié (10) a des propriétés élastiques supplémentaires selon une direction qui n'est pas perpendiculaire à ladite direction d'au moins une partie de ladite pluralité de fentes (18), **caractérisé en ce que** ladite première couche de façade non tissée (14) a été étirée avec striction et est, par suite, dilatable dans une direction parallèle auxdites fentes (18).

7. Stratifié (10) élastique, fibreux, non tissé selon la revendication 6, qui comprend en outre une seconde couche de façade non tissée (16) fixée à une surface de ladite couche de substrat élastique (12) opposée à ladite première couche de façade non tissée (14).

8. Stratifié (10) élastique, fibreux, non tissé selon la revendication 6, dans lequel lesdites fentes (18) sont selon un motif en quinconce avec zones de recouvrement.

9. Stratifié (10) élastique, fibreux, non tissé selon la revendication 6, dans lequel ledit stratifié (10) forme au moins une partie d'un produit absorbant d'hygiène intime.

10. Stratifié (10) élastique, fibreux, non tissé selon la revendication 6, dans lequel ledit stratifié (10) se présente sous la forme d'un champ médical.

11. Stratifié (10) élastique, fibreux, non tissé selon la revendication 6, dans lequel ledit stratifié (10) forme au moins une partie d'un article d'habillement.

12. Stratifié (10) élastique, fibreux, non tissé selon la revendication 7, dans lequel ladite seconde couche de façade non tissée (16) a été étirée avec striction et est, par suite, dilatable dans une direction parallèle auxdites fentes (18).

13. Stratifié (10) élastique, fibreux, non tissé selon la revendication 12, dans lequel ladite couche de substrat élastique (12) comprend une première série de fils élastiques qui sont créés selon une direction telle que l'angle compris entre l'axe longitudinal des fentes et l'axe longitudinal des fils élastiques est compris entre 60° et 120°, et une seconde pluralité de fils élastiques qui sont parallèles auxdites fentes (18).
